# EUROPEAN PATENT SPECIFICATION

(11) **EP 1 732 557 B1**
(45) Date of publication and mention of the grant of the patent: **28.10.2009**
(21) Application number: 05717127.4
(22) Date of filing: 23.03.2005
(51) Int. Cl.: A61K 31/496, A61P 25/14, A61P 25/16, A61K 31/00

(54) **1-[2H-1-BENZOPYRAN-2-ONE-8-YL]-PIPERAZINE DERIVATIVES FOR THE TREATMENT OF MOVEMENT DISORDERS**
1-[2H-1-BENZOPYRAN-2-ONE-8-YL]- PIPERAZINDERIVATIVE ZUR BEHANDLUNG VON BEWEGUNGSSTÖRUNGEN
DÉRIVÉS DU 1-[2H-1-BENZOPYRAN-2-ONE-8-YL]- PIPERAZINE POUR LE TRAITEMENT DES DÉSORDRES DE MOUVEMENT

(30) Priority: 25.03.2004 EP 04101229; 25.03.2004 US 555959 P
(43) Date of publication of application: 20.12.2006
(73) Proprietor: Solvay Pharmaceuticals B.V., 1381 CP Weesp (NL)
(72) Inventor: BAKKER, Cornelis; c/o IPSI Department, NL-1381 CP Weesp (NL); GLENNON, Jeffrey C.; c/o IPSI Department, NL-1381 CP Weesp (NL); HESSELINK, Mayke B.; c/o IPSI Department, NL-1381 CP Weesp (NL); THAETE, Claudia; c/o IPSI Department, NL-1381 CP Weesp (NL); McCREARY, Andrew; c/o IPSI Department, NL-1381 CP Weesp (NL); VAN SCHARRENBURG, Gustaaf J.; c/o IPSI Department, NL-1381 CP Weesp (NL)
(74) Representative: Verhage, Marinus
(86) International application number: PCT/EP2005/051327
(87) International publication number: WO 2005/092339

(56) References cited:
- EP-A- 0 533 268
- EP-A- 0 650 964
- WO-A-93/13766
- WO-A-97/36893
- US-A1- 2002 156 075
- US-A1- 2004 014 769
- DATABASE WPI Week 2002 Derwent Publications Ltd., London, GB; AN 2002-393727 XP002337215 -& WO 02/18367 A (MEIJI SEIKA KAISHA, LTD; KIKUCHI, CHIKA; KOYAMA, MASAO; FUJI, KAZUYUKI) 7 March 2002 (2002-03-07)

## Description

The invention relates to a novel use of known 1-[2H-1-benzopyran-2-one-8-yl]-piperazine derivatives, broad spectrum 5-HT receptor binding compounds, having amongst other functional serotonin receptor activities, potent 5-HT_{1A} receptor agonistic activity, 5-HT_{1D} receptor antagonistic activity and 5-HT₇ receptor agonistic activity. In embodiments of the invention specific compounds disclosed herein are used for the manufacture of medicaments for treating, ameliorating or preventing movement disorders, in particular epilepsy.

Movement disorders are neurological disturbances involving one or more muscles or muscle groups, and include Parkinson's disease, Huntington's Chorea, progressive supranuclear palsy, Wilson's disease, Tourette's syndrome, epilepsy and various chronic tremors, including essential tremor, tics and dystonias. Different clinically observed movement disorders can often be traced to the same or similar brain areas. Abnormalities of basal ganglia for instance, are postulated as a causative factor in diverse movement disorders.

Patients with movement disorders are often subjected to neurosurgery: invasive, irreversible, and not curative in many cases. Drug therapy in movement disorders leaves much to be desired. Many of the currently used drugs have severe side effects. The β-blocker propranolol for instance, often prescribed to patients with tremors, causes significant cardiovascular side effects. Patients with tic disorders are frequently treated with dopamine antagonists: effective drugs, but unfortunately also characterized by many side effects, including (sic) other movement disorders similar to Parkinsonism. A pertinent phenomenon with drug therapy in movement disorders is resistance to drug therapy. This is known to occur with 20% of patients with epilepsy, and an even larger percentage of patients with Parkinson's disease with epilepsy, and an even larger percentage of patients with Parkinson's disease become resistant to L-dopa therapy. Drug resistant tremors can Include resting tremors (e.g. in Parkinson's disease), and action tremors, including essential tremor, multiple sclerosis tremors, post traumatic tremors, post hemiplegic tremors (also known as post stroke spasticity), writing tremors and epilepsy.

It has been known for a long time that the potent and selective 5-HT_{1A} agonist 8-OH-DPAT [8-hydroxy-2-(di-n-propylamino)tetralin] is capable of antagonizing haloperidol induced catalepsy in rats and of attenuating neuroleptic induced dystonia in nonhuman primates (J.M. Liebman et al., Psychopharmacology, 97, 456, 1989). These findings have triggered research directed at compounds with a combination of dopamine-D₂ antagonism and 5-HT_{1A} receptor agonism (see WO 97/36893 and R.W. Feenstra et al., Bioorganic & Medicinal Chemistry Letters, 11, 2345-2349, 2001). Drug-induced effects on motoric systems can not be compared with movement disorders, and up to recent times the state of the art did not contain any incentives towards a possible efficacy of 5-HT_{1A} agonists in movement disorders.

Then in patent application US 2002/0156075 it was suggested that compounds which possess serotonin 5-HT_{1A} agonist activity may be useful for the prevention and/or treatment of a number of acute and chronic conditions, including epilepsy. For the latter no experimental evidence was given. In order to check whether or not this suggestion of anti-epileptic activity could be substantiated, several 5-HT_{1A} agonists were tested for their anticonvulsant activity in DBA/2 mice, an animal model predictive for anti-epileptic activity. Among others, tested were the potent and selective 5-HT_{1A} agonists flesinoxan and 8-OH-DPAT as well as the mono hydrochloric acid mono hydrate of 3-amino-8-(1-piperazinyl)-2H-1-benzopyran-2-one (hereafter named 'compound 1'), a broad spectrum 5-HT receptor binding compound, having amongst other functional serotonin receptor activities, potent 5-HT_{1A}-agonistic as well as 5-HT_{1D}-antagonistic activity.

As more or less expected, the compounds were found active in the test for anti - epileptic activity. But surprisingly, the non-selective 5-HT_{1A}-agonist 'compound 1' was found to be at least ten times more potent than the very selective 5-HT_{1A}-agonists flesinoxan and 8-OH-DPAT (*ED₅₀ values of 0.66 mg*/*kg for compound 1 versus and 7.74 mg*/*kg and 15 mg*/*kg for flesinoxan and 8-OH-DPAT respectively, see below*). This was surprising because in other in vivo tests specific for 5-HT_{1A} receptor agonism the three compounds are nearly equipotent. In the 'lower lip retraction' for instance, ED₅₀-values are 0.12 mg/kg for compound 1 versus 0.22 mg/kg for flesinoxan (*see below*). Taken together, these data strongly suggested the presence of a non-5-HT_{1A} agonistic mechanism inherent in compound 1's mechanism of action in anti-seizure activity.

Compound 1 was known to be a 5-HT_{1A} agonist and 5-HT_{1D} antagonist (EP 0 650 964). Extensive receptor binding studies learned that apart from its nanomolar affinities for 5-HT_{1A} and 5-HT1_{D} receptors, the compound also has a nanomolar affinity for 5-HT₇ receptors (*see binding profile, below*).

Surprisingly, interaction studies learned that both the selective 5-HT_{1D} agonist sumatriptan as well as the selective 5-HT₇ antagonist SB 258741 partly antagonized the anti-epileptic activity of compound 1 (see below), clearly indicating that these serotonin receptor subtypes are involved in seizures. Hitherto no links between 5-HT_{1D} antagonism and/or 5-HT₇ agonism and epilepsy have been established.

Thus, compound 1 and its analogues are compounds with a unique combination of pharmacological activities: 5-HT_{1A} receptor agonistic activity, 5-HT_{1D} receptor antagonistic activity and 5-HT₇ receptor agonistic activity, making them of more value for use in the treatment of epilepsy than compounds like flesinoxan and 8-OH-DPAT, which are extremely selective 5-HT_{1A} agonists only.

Based on the results of the interaction studies described above (*and of which experimental detail are given below*), it is also likely that compounds having the combination of 5-HT_{1D} receptor antagonistic activity and 5-HT₇ receptor agonistic activity are of value when used in the treatment of epilepsy. The same is true for compounds that are either 5-HT_{1D} receptor antagonists or 5-HT₇ receptor agonists.

The compounds are devoid of sedative effects when given in dosages of up to 100 mg/kg *p*.*o*., and were also shown to be highly active as inducers of growth factors. The latter activity is indicative of neuroprotective effects and improvement of brain plasticity required for neuroregeneration. It was also found that the compounds of the invention are active in experimental animal models with predictive value for activity against the symptoms of Parkinson's disease in particular and dyskinesias in general. Moreover, the compounds when given orally show a good bioavailability, which results in high potency and long duration of action.

The present invention describes drugs for the therapy of movement disorders, in particular epilepsy, which have a mechanism of action different from that of drugs currently on the market, give rise to fewer side-effects, and are less prone to develop resistance in patients, in particular in patients resistant to anti-epileptic drug (AED) therapy.

1-[2H-1-Benzopyran-2-one-8-yl]-piperazine derivatives, broad spectrum 5-HT receptor binding compounds (*see receptor binding profile, below*), having amongst other functional serotonin receptor activities, potent 5-HT_{1A}-agonistic as well as 5-HT_{1D}-antagonistic activity, were originally developed as antidepressants (EP 0 650 964). The presence of 5-HT_{1D} antagonism is thought to be of therapeutic value. 5-HT_{1D} receptors are located presynaptically on the nerve terminal and have a negative modulatory influence on the release of 5-HT. Therefore, blockade of these receptors enhances the release of 5-HT from its terminals. The additional presence of presynaptic 5-HT_{1D} antagonism will result in a similar effect as observed after administration of 5-HT reuptake inhibitors. When 5-HT_{1D} antagonism is combined with 5-HT_{1A} agonism the later activity is strengthened.

Pharmaceutically acceptable salts may be obtained using standard procedures well known in the art, for example by mixing a compound of the present invention with a suitable acid, for instance an inorganic acid such as hydrochloric acid, or with an organic acid. The active compounds and their salts can be processed to compositions by means of standard methods, for example pills, tablets, coated tablets, capsules, powders and injection liquids, using auxiliary substances such as liquid and solid carrier materials.

The invention relates to the compound 3-amino-8-(1-piperazinyl)-2H-1-benzopyran-2-one and the salts thereof, i.e. the compound of formula (2):

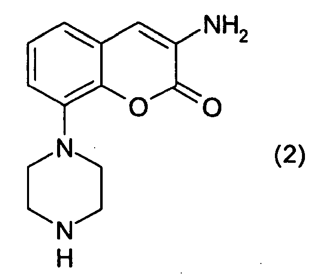

Especially preferred is the mono hydrochloric acid mono hydrate of the compound with formula (2), hereafter referred to as 'Compound 1'.

The compounds of the invention are active at doses in the range of 0.1 - 100 mg/kg after oral administration, and their unique pharmacological profile makes them particularly useful in the treatment of movement disorders, including Parkinson's disease, Huntington's Chorea, progressive supranuclear palsy, Wilson's disease, Tourette's syndrome, symptomatic and non-symptomatic epilepsy, seizures, including refractory seizures and post-stroke seizures and other electroconvulsive disorders, various chronic tremors, including essential tremor, tics and dystonias.

Within the context of this document, below more detailed descriptions are given, of the disorders listed above.

**Parkinson's disease** is a movement disorder of increasing occurrence in aging populations. It is a disabling disease affecting approximately 1% of the population over the age of 60, and the cumulative risk of an individual developing it, is about 1 In 40. Symptoms include pronounced trem or of the extremities, bradikynesia, rigidity and postural change. Parkinson's disease is a progressive disorder which can begin with mild limb stiffness and infrequent tremors and progresses over a period of ten years or more to frequent tremors and memory impairment, to incontrollable tremors and dementia.

**Huntington's Chorea** is a genetically inherited disorder characterized by neurological as well as psychiatric features. Most cases de velop when people are in their forties or fifties. The disease usually begins with neurological or mental status changes. Neurological symptoms may include chorea, a series of movements that is dance-like, jerky, brief, and moves from one part of the body to another. Other symptoms can be clumsiness, fidgetiness and jumpine ss, as well as facial movements, in particular of the jaw. Often difficulties with walking and posture are encountered. Psychiatric symptoms may present as paranoia, confusion or personality changes. As the diseases progresses, a significant dementia devel ops.

**Progressive supranuclear palsy** is a movement disorder in which patients have significant difficulty moving their eyes vertically (up and down) initially, followed by all eye movements become limited (opthalmoplegia). Patient s are prone to develop dementia, rigidity, bradykinesia (slow movements) and a prop ensity to fall.

**Wlison's disease** is a disease involving the nervous system as well as the liver. Neurological symptoms include tremors, in -coordination, falling, slurred speech, stiffness and seizures. Psychiatric problems can occur and patients can develop severe liver damage if this affliction is untreated.

**Tourette's syndrome** is a tic disorder (see below) which begins in childhood or adolescence, and is considerably more common In males. Both multiple motor tics as well as vocal tics are present. Tics may change from involvement of one body part to another, and the disease is characterized by periods with a minimal activity and other times when some patients have difficulty functioning. Tourette' s syndrome is often accompanied by other neurobehavioral difficulties such as attention deficit hyperactivity disorder (ADHD), attention deficit disorder (ADD) or obsessive compulsive behaviour (OCD).

**Epilepsy** is the most common serious neurological disorder, estimated to affect between one half and one percent of the general population. Epilepsy is characterized by recurrent seizures resulting from a sudden burst of electrical energy in the brain. The electrical discharge of brain cells causes a change in a person's consciousness, movement and/or sensations. In humans, epilepsies are separated into two forms: symptomatic and non -symptomatic. Symptomatic epilepsy is a seizure disorder related to a known cause such as metabolic disease, brain malformations, or brain tumors. In these cases, seizures presumably occur because of a very abnormal focus in the brain. Non-symptomatic epilepsies are defined when no structural or metabolic lesions are recognized and the patients have no other neurological findings between seizures. This latter group of patients is more likely to have a primary neuronal hyperexcitability that is not caused by metabolic, developmental or structural lesions.
Within the context of this description, "epilepsy" embraces (1) focal epilepsies including benign occipital epilepsy, benign rolandic epilepsy, frontal lobe epilepsy, occipital lobe epilepsy, medial temporal lobe epilepsy and parietal lobe epilepsy; (2) generalized idiopathic epilepsies including benign myoclonic epilepsy in Infants, juvenile myoclonic epilepsy, childhood absence epilepsy, juvenile absence epilepsy and epilepsy with generalized tonic clonic seizures In childhood; (3) generalized symptomatic epilepsies including infantile spasm (West syndrome), Lennox-Gastaut syndrome and progressive myoclonus epilepsies, and; (4) unclassified epilepsies including refractory epilepsy, post-stroke epilepsy, febrile fits, epilepsy with continuous spike and waves in slow wave sleep, Landau Kleffner syndrome, Rasmussen's syndrome and epilepsy and inborn errors of metabolism.

**Tremors** are characterized by abnormal, involuntary movements. An essential tremor is maximal when the afflicted body part (often arm or hand) is being used. A resting tremor is common in Parkinson's disease, and is maxima I when the extremities are at rest. Resting tremors usually subside when the patient attempts fine movement.

**Tic disorders** are very rapid, short lived stereotyped repeated movements. The more common tics involve the motor systems, or are vocal in nature. Motor tics often involve the eyelids, eyebrows or other facial muscles, as well as the upper limbs. Vocal tics may involve grunting, throat clearing, coughing or cursing. The best known tic disorder is Tourette's syndrome, but tics may also be associated with head injury, carbon monoxide poisoning, stroke, drug (ab)use and mental retardation.

**Dystonias** are involuntary movement disorders characterized by continued muscular contractions which can result in twisted contorted postures involving the body or limbs. Causes of dystonia include biochemical abnormalities, degenerative disorders, psychiatric dysfunction, toxins, drugs and central trauma. Particular dystonias include spasmodic torticolis (a syndrome involving involuntary turning of the neck to one side) blepharospasm (involuntary movement involving intermittent forceful closure of the eyelids) and writer's cramp (a cramping abnormal posture which develops when one is writing, or performing other actions with the hands. Symptoms may progress to involve the arm and shoulder). For the treatment of dystonias neurosurgical procedures are often preferred over drug therapy.

### PHARMACEUTICAL PREPARATIONS

The compounds of the invention can be brought into forms suitable for administration by means of usual processes using auxiliary substances such as liquid or solid carrier material. The pharmaceutical compositions of the invention may be administered enterally, orally, parenterally (intramuscularly or intravenously), rectally or locally (topically). They can be administered in the form of solutions, powders, tablets, capsules (including microcapsules), ointments (creams or gel) or suppositories. Suitable excipients for such formulations are the pharmaceutically customary liquid or solid fillers and extenders, solvents, emulsifiers, lubricants, flavorings, colorings and/or buffer substances. Frequently used auxiliary substances which may be mentioned are magnesium carbonate, titanium dioxide, lactose, mannitol and other sugars or sugar alcohols, talc, lactoprotein, gelatin, starch, cellulose and its derivatives, animal and vegetable oils such as fish liver oil, sunflower, groundnut or sesame oil, polyethylene glycol and solvents such as, for example, sterile water and mono- or polyhydric alcohols such as glycerol.

Types of pharmaceutical compositions that may be used include tablets, chewable tablets, capsules, solutions, parenteral solutions, suppositories, suspensions, and other types disclosed herein or apparent to a person skilled in the art from the specification and general knowledge in the art. In embodiments of the invention, a pharmaceutical pack or kit is provided comprising one or more containers filled with one or more of the ingredients of a pharmaceutical composition of the invention. Associated with such container(s) can be various written materials such as instructions for use, or a notice in the form prescribed by a governmental agency regulating the manufacture, use or sale of pharmaceuticals products, which notice reflects approval by the agency of manufacture, use, or sale for human or veterinary administration.

### PHARMACOLOGICAL METHODS

### ANTI-EPILEPTIC ACTIVITY: ANTICONVULSANT ACTIVITY IN DBA/2 MICE

### Animals

DBA/2 mice weighing 6-12 g (22-26 days old; either sex, Harlan Italy Correzzana, Milano, Italy) are housed in groups of 8-10 under a 12-h light/dark cycle (lights on at 7:00 a.m.) with food and water available *ad libitum.* The experimental protocol is approved by University of Catanzaro Ethical Committee. All procedures are in compliance with the Nationals Institutes of Health Guide for Care and Use of Laboratory Animals (Publication No. 85-23, revised 1985) and European Communities Council Directive of 24 November 1986 (86/609 EEC).

### Audiogenic seizures in DBA/2 mice

The experiments are performed according to the method previously described by De Sarro et al. (1984), Eur.J.Pharmacol., 104, 55-60. Compounds of the invention or vehicle (controls) are given intraperitoneally (*i.p*.) and orally (*p*.*o*.) as freshly sonicated solutions in methylcellulose 1%. For the *i*.*p*. treatment compounds of the invention are given at doses of 0.1, 0.3, 3, 10, 15, 20 and 30 mg/kg; pretreatment time at 30 and 60 min., For oral treatment, all animals are pretreated either at 30 or 60 min using the compounds of the invention at doses of 0.1, 0.3, 3, 6 and 10 mg/kg *p*.*o*. At least 10 mice per dose are used. After pretreatment time, each mouse is placed under a hemispheric perspex dome (58 cm in diameter) and 1 min is allowed for habituation and assessment of locomotor activity. Animals are challenged with a 12-16 kHz sinusoidal tone at 109 dB. Seizure response is assessed by two independent observers. The sound evoked behaviour is coded using the following scale: 0= no response, 1= wild running, 2= clonus, 3= tonic flexor and/or extensor; 4= respiratory arrest, on the basis of the concordant opinion of the observers. Sound stimulus is applied for 60s, but it is interrupted earlier, when the observed animal shows tonic extensor seizure. The maximum response is recorded for each animal. Rectal temperature is recorded immediately prior to auditory testing using an Elektrolaboratoriet thermometer type T.E.3. Behavioral changes are observed during the period between drug administration and audit ory testing. In these experiments separate vehicle-treated and drug-treated groups are used at each pretreatment time (15 min-60 min). Percentage of mice showing wild running, clonic or tonic phase and the duration of anticonvulsant activity are compared to control group and statistically analyzed.

### Statistical analysis

Statistical comparison between groups of control and drug -treated DBA/2 mice is made using Fisher's exact probability test (incidence of the seizure phases). The percentage incidence of each phase of the audiogenic seizure is determined for each drug. These values are plotted against the corresponding doses by a computer construction of the dose-effect curves for calculation of ED₅₀ (with 95% confidence limits). The ED₅₀ values for each compound are calculated using a computer program of the method of Litchfield and Wilcoxon (1949). At least 32 animals are used to calculate each ED₅₀ value.

### Drugs

For systemic injections and oral administrations, all compounds are given intraperitoneally (0.1 ml/10 g of body weight of the mouse) as a freshly prepared solution in methylcellulose 1%. In order to avoid the light sensitivity, weighing and handling are carried out under sodium vapor lamps and the substances are protected from light during the experiments.

### NEUROPROTECTIVE ACTIVITY: INDUCTION OF GROWTH FACTORS

Compounds of the invention (3 mg/kg, *p*.*o*.) or vehicle are administered once daily during a period of 3 weeks (n=8 animals per treatment group). 24 hours after the last dose the animals are sacrificed (using CO₂/O₂ anesthesia), the brains are removed and the dissected. RNA is extracted from the individual brain samples and induction of the growth factors BDNF and GDNF is determined by quantitative PCR. Total RNA is isolated with the Trizol method (Invitrogen) from the brain pieces. cDNA is made starting with 2 µg of total RNA (pretreated for 30 min with DNAse (Ambion) in first strand buffer) using the reverse transcriptase Superscript II (Invitrogen). Quantification of mDNA by real time PCR makes use of the observation that the early cycles of PCR are characterized by an exponential increase in target amplification. The accumulation of PCR product is measured using Sybergreen II. Primers are designed using the software package Primer Express (Applied Blosystems). Expression levels of the housekeeping genes omithine decarboxylase (ODC_ex8) and alpha tubulin (TUBA) used for normalization and as control for good cDNA synthesis.

### ANTI-PARKINSONIAN ACTIVITY: TURNING EXPERIMENTS IN ANIMALS WITH UNILATERAL 6-HYDROXYDOPAMINE LESIONS OF THE SUBSTANTIA NIGRA COMPACTA

### Animals

Male rats (Wistar, Harlan, Netherlands; 400-500g at time of experiment) are housed in a temperature (20-21±2°C) and humidity controlled environment and receive water *ad libitum* except during experimental sessions. Food is restricted to approximately 15g per rat per day. A 12-hour light-dark cycle (lights on 07.00-19.00 hour) is used. All experimental procedures are conducted in accordance with Dutch law and conform to local animal care and use committee stipulations.

### Surgery

Unilateral 6-hydroxydopamine (6-OHDA) lesions of the *substantia nigra zona compacta* are performed using a stereotaxic procedure. One hour prior to surgery, desmethyl-imipramine (20 mg/kg, *i*.*p*.) is administer ed to protect noradrenergic neurons. Rats are anaesthetized with a 3% halothane + 0.8 l/min N₂O + 0.8 l/min O₂ -gas mixture at 1013 mbar. During surgery the gas mixture is adjusted to 1.75 - 2% halothane, 0.6 l/min N₂O and 0.6 l/min O₂. The incisor bar of the stereotaxic instrument (Kopf, California, USA) iss set at -3.3 mm, a burr hole was drilled over the substantia nigra pars compacta and 3 µl of a 6-OHDA solution (3.33 mg/ml) is injected (flow rate=0.75 µl/min; the needle is left in place for 4 minutes prior to withdrawal). Coordinates for this procedure are: anterior posterior +3.2 mm from the interaural line; medial/lateral +1.8 mm from the midline and ventral -8.2 from the skull surface. Animals are allowed to recover for approximately 2 weeks prior to testing. Good turning rats are defined as those which elicited at least 20 contralateral turns following amphetamine (2.5 mg/kg sc) in the 5 min time epoch beginning 25 min after administration and a mean of at least 20 contralateral turns recorded over a 30 min period after administration of apomorphine (0.25mg/kg *s*.*c*.). Regular testing with apomorphine (0.1 or 0.25 mg/kg *s*.*c*.) is carried out to ensure the reliability of the animals in this procedure.

### Apparatus

Eight commercially available (TSE systems Bad Homburg, Germany) 'rotameter' units (transparent plastic bowls; 57 x 55 x 52 cm) are used for testing. The rats are harnessed and tethered to a rotation sensor interfaced to an IBM compatible personal computer (using the TSE Rotameter Software v. 1.11 , TSE systems Bad Homburg, Germany) which registers clockwise or counterclockwise movement. An internal software rotation filter of 10 is used.

### Protocol

Following statistical randomization of the treatment groups rats are pretreated with compounds of the invention (0.1-3 mg/kg *p*.*o*.) or vehicle (2ml/kg) and placed in the rotameters the contralateral rotational behaviour is then measured. In further studies the effects of L-DOPA (1-10 mg/kg *p*.*o*.) are assessed on contralateral rotations. The peripheral decarboxylase inhibitor benserazide (30 mg/kg *i*.*p*.) can be used. In combination studies a range of L-DOPA (1-10) doses and doses of compounds of the invention (0.1-3 mg/kg *p*.*o*.) can be combined.

### RECEPTOR BINDING EXPERIMENTS

Receptor binding data were obtained by CEREP (128, rue Danton, 92500 Rueil-Malmaison, France) or at Solvay Pharmaceuticals B.V., using well documented standard procedures. Affinity for 5-HT1A receptors for instance, was determined by testing the ability of the compounds of the invention to displace [³H]-2-(di-n-propylamino)-8-hydroxytetralin ([³H]-8-OH-DPAT) from its specific binding sites in rat frontal cortex homogenates. This test is based on the method described by Gozlan et al. (Nature, 305, (1983), pages 140-142).

### IN VIVO 5-HT_{1A} AGONISM: LOWER LIP RETRACTION (LLR)

Lower lip retraction was measured according to the method described by Berendsen et al., (Pharmacol. Biochem. Behav. 33, (1989), 821-827).

### DOSE

The affinity of the compounds of the invention for serotonin receptors was determined as described above. From the binding affinity measured for a given compound of formula (1), one can estimate a theoretical lowest effective dose. At a concentration of the compound equal to twice the measured Kᵣ-value, 100% of the receptors are likely to be occupied by the compound. Converting that concentration to mg of compound per kg of patient yields a theoretical lowest effective dose, assuming ideal bioavailability. Pharmacokinetic, pharmacodynamic, and other considerations may alter the dose actually administered to a higher or lower value. The dosage expediently administered is 0.001 - 1000 mg/kg, preferably 0.1-100 mg/kg of patient's bodyweight.

### EXAMPLE I. MATERIALS AND METHODS

All reactions involving moisture sensitive compounds or conditions were carried out under an anhydrous nitrogen atmosphere. Reactions were monitored by using thin - layer chromatography (TLC) on silica coated plastic sheets (Merck precoated silica gel 60 F254) with the indicated eluent. Spots were visualised by UV light (254 nm) or I₂. Flash chromatography refers to purification using the indicated eluent and Acros silica gel (0.030-0.075 mm). Nuclear magnetic resonance spectra (¹H NMR and ¹³C NMR, APT) were determined in the indicated solvent with tetramethylsilane as an internal standard. Chemical shifts are given in ppm (δ scale) downfield from tetra-methylsilane. Coupling constants *J* are given in hertz (Hz). Peakshapes in the NMR spectra are indicated with the symbols 'q' (quartet), 'dq' (double quartet), 't' (triplet), 'dt' (double triplet), 'd' (doublet), 'dd' (double doublet), 's' (singlet), 'bs' (broad singlet) and 'm' (multiplet). Melting points were recorded on a Büchi B -545 melting point apparatus. Yields refer to isolated pure products.

### NUCLEAR MAGNETIC RESONANCE (NMR) SPECTROSCOPY

NMR spectra were recorded on a Bruker AM400 spectrometer, or a Varian VXR400S spectrometer. Chemical shifts (δ) were reported in ppm downfield from TMS as internal standard. A sample of 10-50 mg was dissolved in a deuterated solvent, usually CDCl₃ or a DMSO-d6/CDCl₃ (4:1 v/v) mixture). The solvent was selected to ensure complete dissolution of the sample. The free induction decays were generally obtained at room temperature under the following conditions:
- Digital resolution: : 0.2 Hz
- Sweep width: : 18 ppm
- Pulse width: : 20 degrees
- Pulse repetition time: : 4.5 sec or longer if required for complete relaxation
- Carrier frequency: : 6.0 ppm
- Number of acquisitions: : 128 or more if necessary. The C-13 satellite signals at 0.5% signal intensity should be clearly visible.

NMR was used as method for determining relative contents.

### TITRIMETRY (CHLORIDE AND WATER DETERMINATIONS)

For potentiometric titrations, a Metrohm model E636 (Switzerland) was used.

Potentiometric chloride determinations were used in this syntheses to determine chloride. The titration was performed with a combined silver electrode and silver nitrate titrant. The method is specific for chloride because it can distinguish chloride from iodide and bromine on basis of different electrode potentials.

Voltametric titrations for the determination according to Karl Fisher were performed using a Metrohm 633KF (Metrohm, Switzerland) apparatus according to the USP method.

### EXAMPLE II. SYNTHESIS OF 3-AMINO-8-(1-PIPERAZINYL)-2H-1-BENZOPYRAN-2-ONE AND ITS MONOHYDROCHLORIC ACID MONOHYDRATE

### (Compound 1)

### STEP 1: NITRATION

The first step was the nitration of 5-bromo-2-hydroxybenzaldehyde **(1*)** yielding 5-bromo-2-hydroxy-3-nitrobenzaldehyde **(2*):**

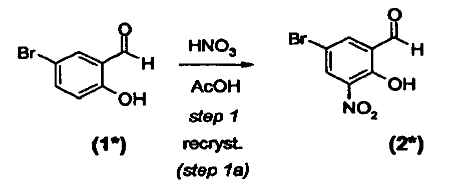

A solution of 1.0 mol of 5-bromo-2-hydroxybenzaldehyde **(1*)** in 3.75 litres acetic acid (98%) was formed on heating the mixture to about 60°C. 1.5 mol of concentrated nitric acid (137 g = 97 ml) was added slowly in approximately 1 hour. After the completion of the addition stirring was continued at 65°C for a further 10 minutes. The solution was then cooled to 45°C, and the product was precipitated by the addition of 4 litres of water. After stirring for at least 3 hours the product was collected on a filter and washed with water until the pH of the mother liquor was approximately 6. The material is dried as much as possible by centrifugation. The crude product was dissolved in 800 ml acetone under refluxing and stirring. 400 ml acetone was removed by distillation. After cooling to 20°C, the mixture was stirred for 3 hours. The precipitate was collected on a filter and washed with petroleum ether 40-65°C. The solid was dried overnight in an air stream at 40°C. Finally, the crude **(2*)** was recrystallized from acetone to yield an end product with a purity of 98% as shown by NMR analysis.

5-bromo-2-hydroxybenzaldehyde **(1*)** was identified by its characteristic chemical shift δ 9.84 ppm; 5-bromo-2-hydroxy-3-nitrobenzaldehyde **(2*)** had a characteristic chemical shift of δ 10.4 ppm.

The overall yield of this step was approximately 60% (crude on crude).

### STEP 2: ERLENMEYER CONDENSATION

The second step was the Erlenmeyer condensation of 5-bromo-2-hydroxy-3-nitrobenzaldehyde **(2*)** with N-acetyl-glycine to yield N-(6-bromo-8-nitro-2-oxo-2H-1-benzopyran-3-yl)acetamide **(3*).**

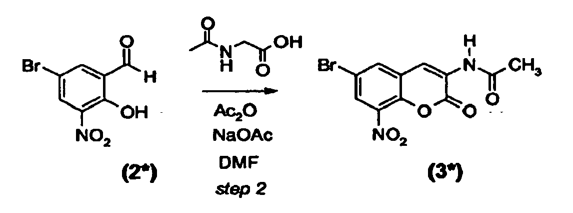

To a mixture of 1.0 mol of 5-bromo-2-hydroxy-3-nitrobenzaldehyde **(2*),** 1.0 mol of N-acetylglycine and 1.0 mol of anhydrous sodium acetate, 800 ml of N -methyl-2-pyrrolidone are added. The mixture was stirred and heated to 50 °C. Then 2.2 mol of acetic anhydride was run into the reaction vessel in approximately 30 minutes. The reaction mixture was heated to 100°C. During heating the reacting mixture became homogeneous for a while; shortly afterwards a solid was formed, making stirring troublesome. After heating at 100°C for 4 hours, the mixture was cooled to 80°C and 1,100 ml of acetic acid (98%) was added. Thereafter stirring of the mixture was easy. Next, the mixture was cooled to room temperature, and stirred for 60 minutes. The precipitate was collected on a filter and washed twice with 625 ml acetic acid (80%), five times with 900 ml water, and once with 300 ml acetone. The product was dried in an air stream at 40°C for 24 hours, and had a purity of 98% as shown by NMR analysis.

5-bromo-2-hydroxy-3-nitrobenzaldehyde **(2*)** had a characteristic shift of δ 10.4 ppm; the characteristic chemical shift of N -(6-bromo-8-nitro-2-oxo-2H-1-benzopyran-3-yl)acetamide **(3*)** was δ 8.72 ppm

The overall yield of this step was approximately 80% (crude on crude).

### STEP 3: REDUCTION

The third step was the catalytic hydrogenation of N-(6-bromo-8-nitro-2-oxo-2H-1-benzopyran-3-yl)acetamide **(3*)** to N-(8-amino-2-oxo-2H-1-benzopyran-3-yl)-acetamide **(4*).**

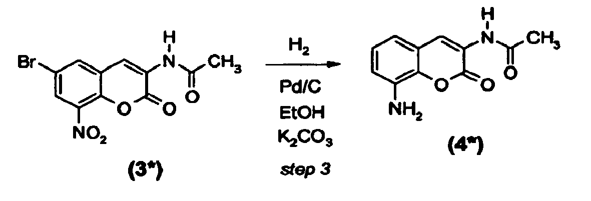

A mixture of 1.0 mol of N-(6-bromo-8-nitro-2-oxo-2H-1-benzopyran-3-yl)acetamide **(3*),** 50 g of 10% palladium on carbon paste (containing 61% water), 1.0 mol of potassium carbonate and 15 litre of ethanol was heated to 60°C. At this temperature the starting material was reduced with hydrogen at an overpressure of 4 bar at 1400 rpm. After completion of the reaction (1 hour), the catalyst was removed by filtration using filteraid, and washed with 4.5 litre methyl ethyl ketone (MEK). The filtrate was concentrated to 2 litre, and 2.3 litre of MEK was added In order to change the solvent from ethanol to MEK, 2 litre of the solvent mixture was distilled off at normal pressure and 2 litre of MEK was added. This was repeated 4 times. Then 5 litre of MEK and 2.6 litre of water were added and the mixture was stirred. The layers were separated. The upper later was concentrated at normal pressure to approximately 3.5 litre. The residue was cooled to 25°C. During this cooling the product crystallized. Then the mixture was cooled to -10°C and stirred for two hours. The solid was filtered and washed three times with 800 ml hexane. The product was dried (50°C, 20 cm Hg, N₂) until constant weight.
N-(6-bromo-8-nitro-2-oxo-2H-1-benzopyran-3-yl)acetamide **(3*)** had a characteristic chemical shift of δ 8.72 ppm; that of N-(8-amino-2-oxo-2H-1-benzo-pyran-3-yl)-acetamide **(4)** was δ 8.55 ppm

The overall yield of this step was approximately 70% (crude on crude).

### STEP 4: CONSTRUCTION OF PIPERAZINE RING SYSTEM

Step 4 was the alkylation of N-(8-amino-2-oxo-2H-1-benzopyran-3-yl)-acetamide **(4*)** with bis-chloroethylamine yielding N-(8-(1-piperazinyl)-2-oxo-2H-1-benzopyran-3-yl-) acetamide **(5*).**

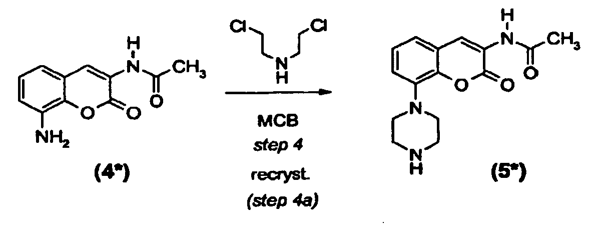

A mixture of 2.5 litre monochlorobenzene, 1.0 mol of N-(8-amino-2-oxo-2H-1-benzopyran-3-yl)-acetamide **(4*)** and 1.2 mol bischloroethylamine hydrochloride was heated to reflux under nitrogen. Part of the monochlorobenzene (0.5 litre) was distilled off. This mixture was refluxed for 10 days. The reaction was followed by HPLC. After the reaction, the mixture was cooled to 20 °C and stirred overnight. The solid product was collected on a filter and washed once with 360 ml monochlorobenzene and 3 times with 360 ml ethanol. The product was dried in vacuum at 50°C.
Half of the crude product was dissolved in 3 litre water. After addition of 18 g of Celite and 50 g of charcoal, the mixture was stirred for 1 hour at room temperature. After filtration the solution was concentrated by distillation of water. In the mean time the second half of the crude product was treated as described above. When the total volume of the combined aqueous solutions was about 1.5 litre, distillation was stopped and the mixture was cooled to room temperature. Then 125 g sodium bicarbonate was added in portions. After stirring for 1.5 hours at 15°C the precipitate formed was collected on a filter. After washing with 360 ml water and 2 times with 270 ml ethanol, the product was dried in vacuum at 50 °C.
N-(8-amino-2-oxo-2H-1-benzo-pyran-3-yl)-acetamide **(4*)** had a characteristic chemical shift of δ 8.55 ppm; that of N-(8-(1-piperazinyl)-2-oxo-2H-1-benzopyran-3-yl-)acetamide **(5*)** was δ 8.57 ppm.

The overall yield of this step was approximately 50% (crude on crude).

### STEP 5: AMIDE HYDROLYSIS

Step 5 was the hydrolysis of the amide function of N-(8-(1-piperazinyl)-2-oxo-2H-1-benzopyran-3-yl-)acetamide **(5*)** using hydrochloric acid. This resulted in the trihydrochloric acid salt of 3-amino-8-(1-piperazinyl)-2H-1-benzopyran-2-one **(6*).**

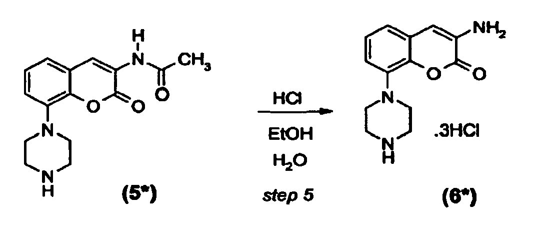

2.9 Litre of concentrated hydrochloric acid was added at room temperature to a suspension of 1.0 mol of N-(8-(1-piperazlnyl)-2-oxo-2H-1-benzopyran-3-yl-) acetamide **(5*)** and 1.4 litre of absolute ethanol in about 10 minutes. During this addition the temperature rose to 40°C. After the addition the mixture was stirred at a temperature of 50°C during 1.5 hours. The mixture was cooled to 20°C and, after crystallisation had started, 1.4 litre of absolute ethanol was added. Then the mixture was stirred for 1 hour at 20°C and for 2 hours at 0°C. The crystals were isolated by filtration and washed twice with 0.6 litre of acetone. The isolated product was dried in vacuum (40°C, 200 mm Hg, N₂, 24 hours).

N-(8-(1-piperazinyl)-2-oxo-2H-1-benzopyran-3-yl-)acetamide **(5*)** had a characteristic chemical shift of δ 8.57 ppm; the trihydrochloric acid salt of 3-amino-8-(1-piperazinyl)-2H-1-benzopyran-2-one **(6*)** had a characterristic chemical shift of 8 6.77 ppm.

The overall yield of this step was approximately 85% (crude on crude).

### STEP 6: PARTIAL NEUTRALISATION

The final step, the sixth, was the partial neutralisation of the trihydrochloric acid salt **(6*)** with sodium bicarbonate to produce the desired product: COMPOUND 1, the mono hydrochloric acid mono hydrate of 3-amino-8-(1-piperazinyl)-2H-1-benzopyran-2-one **(7*)**

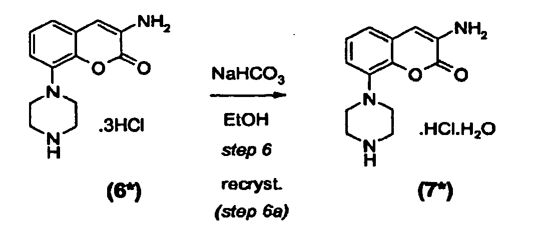

To a suspension of 1.0 mol of the trihydrochloric acid salt **(6*)** in 3.5 litre ethanol a solution of 2.2 mol sodium bicarbonate in 2.8 litre water was added in about 30 minutes. The temperature was between 20°C and 25°C. The suspension was then stirred for 3 hours. The reaction mixture was filtered and subsequently washed with 1.1 litre water, 1.1 litre ethanol and 1.1 litre hexane. The isolated crude product was dried in vacuum (40°C, 200 mm Hg, N₂, 24 hours).

The dried product (1 mol) was dissolved in 9 litre methanol by heating to reflux temperature. The solution did not become completely clear. After cooling to 20 °C the mixture was filtered. 300 ml of water and 150 ml of methanol was added to the filtrate, after which about 3 litre of the solvent mixture was distilled at normal pressure. The complete procedure was repeated with another mol of the dried product. Then the combined fractions wre concentrated to a volume of about 12 litre by distillation. After addition of 6 litre ethanol, 6 litre of the solvent mixture was removed by distillation at normal pressure. The mixture was then cooled to 0°C and stirred for 2 hours. The precipitate was collected on a filter and washed twice with 750 ml acetone. The product was dried under vacuum (40°C, 200 mm Hg, N₂, 24 hours), and thereafter homogenized by milling and, when necessary, by micronizing.

The trihydrochloric acid salt of 3-amino-8-(1-piperazinyl)-2H-1-benzopyran-2-one **(6*)** had a characteristic chemical shift of δ 6.77 ppm; that of the endproduct, Compound 1, was δ 6.7 ppm.

The overall yield of this step was approximately 85% (crude on crude).

Compound 1, the mono hydrochloric acid mono hydrate of 3-amino-8-(1-piperazinyl)-2H-1-benzopyran-2-one, had a molecular formula C₁₃H₁₈ClN₃O₃ and a molecular mass of 299.5. The pure product (99.8%, NMR) was a white to yellowish powder. Its chloride content was 11.7% (mass to mass), as determined by titrimetry. Its water content, determined by Karl Fisher water assay titration, was 6.5% (mass to mass).

### EXAMPLE III: FORMULATION OF COMPOUND 1

**For oral *(p.o.)* administration:** to the desired quantity (0.5-15 mg) of Compound 1 in a glass tube, some glass beads were added and the substance was milled by vortexing for 2 minutes. After addition of 1 ml of a solution of 1% methylcellulose in water, the compound was suspended by vortexing for 10 minutes. For concentrations up and above 1 mg/ml remaining particles in the suspension were further suspended by using an ultrasonic bath.

**For intraperitoneal *(i.p)* administration:** to the desired quantity (0.5-15 mg) of the solid compound 1 in a glass tube, some glass beads were added and the solid was milled by vortexing for 2 minutes. After addition of 1 ml of a solution of 1 % methylcellulose and 5% mannitol in water, the compound was suspended by vortexing for 10 minutes. Finally the pH was adjusted to 7.

### EXAMPLE IV: PHARMACOLOGICAL TESTRESULTS

### ANTIEPILEPTIC ACTIVITY:

### INTRAPERITONEAL ADMINISTRATION OF COMPOUND 1 IN DBA/2 MICE

Compound 1, administered intraperitoneally (*i.p*.) as freshly sonicated solution in DBA/2 mice, showed dose -dependent anticonvulsant properties against audiogenic seizures (*for a description of this method: see above*). In particular, 30 min after *i*.*p*. administration, compound 1 (10, 15, 20 and 30 mg/kg) significantly antagonized the tonic and clonic phase of audiogenic seizures, whereas the lower dose of 3 mg/kg was only able to significantly antagonize the tonic phase. In addition, 60 min. after i.p. administration, compound 1 (10, 15, 20 and 30 mg/kg) significantly antagonized the tonic and clonic phase of audiogenic seizures, whereas compound 1 (3 and 0.3 mg/kg) was only able to significantly antagonize the tonic phase of audiogenic seizures. Other doses not reported above were not able to significantly modify the occurrence of tonic and/or clonic seizures. The respective ED₅₀ values (± 95% confidence limits) are reported in Table 1. At none of the dosages tested mice receiving compound 1 showed any behavioural changes.

### INTRAPERITONEAL ADMINISTRATION OF FLESINOXAN IN DBA/2 MICE

Flesinoxan, administered intraperitoneally (i.p.) as freshly solution (sterile saline) in DBA/2 mice, showed the following effects against audiogenic seizures. In particular, 30 min after i.p. administration, Flesinoxan (0.3, 1, 3, 5 and 7,5 mg/kg) did not significantly antagonized the tonic and clonic phase of audiogenic seizures. On the contrary the doses of 10 and 15 mg/kg were able to significantly (P < 0.01) antagonize the tonic and clonic phase of audiogenic seizures (Table 1). At none of the dosages tested mice receiving flesinoxan showed any behavioural changes.

### INTRAPERITONEAL ADMINISTRATION OF 8-OH-DPAT IN DBA/2 MICE

8-OH-DPAT, administered intraperitoneally (i.p.) as freshly solution (sterile saline) in DBA/2 mice, showed the following behavioural effects against audiogenic seizures. In particular, 30 min after i.p. administration, 8-OH-DPAT (0.3, 1, 3, 10, 15 and 20 mg/kg) did not significantly antagonized the clonic phase of aud iogenic seizures and the doses of 0.3, 1, 3, 10 mg/kg did not significantly antagonized the tonic phase of audiogenic seizures. On the contrary, the doses of 15 and 20 mg/kg were able to significantly (P < 0.01 and P < 0.05) antagonize the tonic phase of audiogenic seizures (Table 1). At none of the dosages tested mice receiving 8-OH-DPAT showed any behavioural changes.

### INTRAPERITONEAL ADMINISTRATION OF SB258741 IN DBA/2 MICE

SB258741, administered intraperitoneally (i.p.) as freshly solution (sterile sall ne) in DBA/2 mice, showed the following effects against audiogenic seizures. In particular, 30 min after i.p. administration, SB258741 (1, 3, 10 and 15 mg/kg) did not significant-ly antagonize the clonic phase of audiogenic seizures. On the contrary the dose of 15 mg/kg was able to significantly (P < 0.01) antagonize the tonic phase of audio-genic seizures (Table 1). At none of the dosages tested mice receiving SB 258741 showed any behavioural changes.

**Table 1: Effects of against audiogenic seizures in DBA/2 mice.**

| | | **ED₅₀ values in mg/kg after i.p. administration** | | |
|---|---|---|---|---|
| | | | | |

| **compound** | **Time¹** | **Tonic extension** | **Clonic seizures** | **Wild running** |
|---|---|---|---|---|
| | | | | |
| comp. 1 | 30 | **0.66** *(0.22-2.01)* | **3.70** *(1.73-7.94)* | **39.04** *(17.54-86.89)* |
| comp. 1 | 60 | **0.26** *(0.07-0.94)* | **1.90** *(0.72-5.08)* | **20.51** *(9.73-43.25)* |
| | | | | |
| flesinoxan | 30 | **7.74** *(5.9-10.13)* | **9.37** *(7.20-12.18)* | **> 15** |
| | | | | |
| 8-OH-DPAT | 30 | **± 15** | **> 20** | **> 20** |
| | | | | |
| SB258741 | 30 | **± 12** | **> 15** | **> 15** |

| | | | | |
|---|---|---|---|---|
| *Time¹ : minutes after drug administration. All data reported above are expressed* as *mg*/*kg i.p. and were calculated according to the method of Litchfield and Wilcoxon (1949). Values in parentheses are* ±*95% confidence limits.* | | | | |

### INTERACTION STUDIES IN DBA/2 MICE: COMPOUND 1 + VEHICLE, SUMATRIPTAN OR SB 258741

Compound 1, administered intraperitoneally (i.p.) as freshly sonicated solution in DBA/2 mice, showed dose-dependent anticonvulsant properties against audiogenic seizures. In particular, 30 min after i.p. administration, Compound 1 (10, 15 and 20 mg/kg) significantly (P < 0.01) antagonized the tonic and clonic phase of audiogenic seizures, whereas the lower dose of 3 mg/kg was only able to significantly (P < 0.01) antagonize the tonic phase. The dose of 0.3 was not able to significantly modify the occurrence of tonic and/or clonic seizures. The pre-treatment with either SB 258741 (3 mg/kg, i.p., 5 min before) or sumatriptan (3 mg/kg, i.p., 5 min before) was able to shift the dose-response curve of COMPOUND 1 to the right. Sumatriptan showed to be more potent than SB 258741. At none of the dosages tested mice receiving COMPOUND 1 + vehicle, COMPOUND 1 + SB 258741 or COMPOUND 1 + sumatriptan, showed any behavioural changes.

**Table 2: Interactions studies: effects of against audiogenic seizures in DBA/2 mice.**

| | **Percentage of animal response** | | | | | |
|---|---|---|---|---|---|---|
| | | | | | | |

| | **Tonic extension** | | | **Clonic seizures** | | |
|---|---|---|---|---|---|---|
| | | | | | | |

| **Dose ¹** | **Cp 1²** | **Cp 1+S³** | **Cp 1+SB⁴** | **Cp 1²** | **Cp 1+S³** | **Cp 1+SB⁴** |
|---|---|---|---|---|---|---|
| | | | | | | |
| **0.3** | **60** | **90** | **90** | **90** | **100** | **100** |
| **3** | **40** | **60** | **50** | **80** | **100** | **90** |
| **10** | **0** | **20** | **10** | **40** | **60** | **50** |
| **15** | **0** | **0** | **0** | **20** | **40** | **30** |
| **20** | **0** | **0** | **0** | **10** | **20** | **20** |

| | | | | | | |
|---|---|---|---|---|---|---|
| Dose¹ = dose of Compound 1 In mg/kg: Cp 1² = Compound 1 + vehicle, Cp 1 + S ³ = Compound 1 + 3 mg/kg (i.p.) sumatriptan, Cp 1 + SB⁴ = Compound 1 + 3 mg kg (i.p.) SB 258741 | | | | | | |

### ANTIEPILEPTIC ACTIVITY:

### ORAL ADMINISTRATION OF COMPOUND 1 IN DBA/2 MICE

Compound 1, administered orally (*p.o.*) as freshly sonicated solution in DBA/2 mice, showed dose-dependent anticonvulsant properties against audiogenic seizures. In particular, 30 min after oral administration, Compound 1 (6 and 10 mg/kg) significantly antagonized the tonic and clonic phase of audiogenic seizures, whereas the lower dose of 3 mg/kg was only able to significantly antagonize the tonic phase. In addition. 60 min. after oral administration Compound 1 (3, 6 and 10 mg/kg) significantly antagonized the tonic and clonic phase of audiogenic seizures, whereas Compound 1 0.3 mg/kg was only able to significantly antagonize the tonic phase of audiogenic seizures. Other doses not reported above were not able to significantly modify the occurrence of tonic and/or clonic seizures. The respective ED₅₀ values (± 95% confidence limits) are reported In Table 3.
At none of the dosages tested mice receiving Compound 1 showed any behavioural changes.

**Table 3: Effects of against audiogenic seizures in DBA/2 mice: oral administration.**

| | | **ED₅₀ values in mg/kg after p.o. administration** | | |
|---|---|---|---|---|
| | | | | |

| **compound** | **Time¹** | **Tonic extension** | **Clonic seizures** | **Wild running** |
|---|---|---|---|---|
| | | | | |
| comp. **1** | 30 | **1.19** *(0.53-2.70)* | 4.91 *(2.11-11.43)* | ND |
| comp. **1** | 60 | **0.27** *(0.07-1.01)* | **1.34** *(0.45-3.98)* | ND |

| | | | | |
|---|---|---|---|---|
| *Time¹: minutes after drug administration. All data reported above are expressed as mg*/*kg i.p. and were calculated according to the method of Litchfield and Wilcoxon (1949). Values In parentheses are* ±*95% confidence limits. ND= not detected*. | | | | |

### INDUCTION OF GROWTH FACTORS

Treatment with Compound 1 increased GDNF and BDNF RNA levels in the thalamus, striatum, prefrontal cortex, nucleus accumbens and hippocampus (table)

**Table 4: regulation of growth factor RNA by treatment with Compound 1 as determined by quantitative PCR. Data are expressed as fold increase compared to vehicle treatment.**

| Brain area | GDNF | BDNF |
|---|---|---|
| thalamus | **2.6 (p<0.001)** | **2.3 (p<0.0001)** |
| striatum | **2.2 (p<0.00005)** | 1.8 (p=0.12) |
| prefrontal cortex | 1.3 (p=0.24) | **2.1 (p<0.0005)** |
| nucleus accumbens | 1.5 (p<0.05) | 5.1 (p<0.05) |
| hippocampus | **1.7 (p<0.001)** | **2.4 (p<0.0001)** |

### ANTI-PARKINSONIAN ACTIVIIY: TURNING EXPERIMENTS IN ANIMALS WITH UNILATERAL 6-HYDROXYDOPAMINE LESIONS OF THE SUBSTANTIA NIGRA COMPACTA

Compound 1 induced a dose-dependent [F(4.35) = 3.87, p<0.01, minimal effective dose of 0.75 mg/kg] rotation contralateral to the side of a 6-OHDA lesion suggesting that the compound could have potential antiparkinsonian properties.

**Table 5. Number of rotations contralateral to the direction of a 6 -OHDA lesion.**

| **Dose (mg/kg p.o.) of compound 1** | **Mean number of turns** |
|---|---|
| | |
| **0** | **-3.3** |
| **0.3** | **20.925** |
| **0.5** | **22.988** |
| **0.75** | **106.35*** |
| **1.0** | **77.1*** |

| | |
|---|---|
| * indicates significantly different to vehicle treated animals (1-way ANOVA followed by post-hoc Dunnett's test). (- indicates ipsilateral rotation). | |

### IN VIVO 5-HT_{1A} AGONISM: LOWER LIP RETRACTION (LLR)

The ED₅₀-values in the 'lower lip retraction' model (see above) were found to be 0.12 mg/kg and 0.22 mg/kg respectively for compound 1 and flesinoxan, 60 minutes after oral administration.

### RECEPTOR BINDING PROFILE OF COMPOUND 1.

The binding data collected in the table below were either obtained by CEREP (128, rue Danton, 92500 Rueil-Malmaison, France) or at Solvay Pharmaceuticals B.V., using well documented standard procedures.

| **receptor** | **S¹** | **radioligand** | **Kᵢ(nM) Compound 1** |
|---|---|---|---|
| | | | |
| 5-HT_{1A} | h | [³H]-8-OH-DPAT | **0. 25** |
| 5-HT_{1B} | r | [¹²⁵I]-cyanopindolol | **2.0** |
| 5-HT_{1D} | b | [³H]-serotonin | **13** |
| 5-HT_{2A} | h | [³H]-ketanserin | **630** |
| 5-HT_{2B} | h | [³H]-LSD | **320** |
| 5-HT_{2C} | h | [¹²⁵I]-DOI | **> 1,000** |
| 5-HT₃ | h | [³H]-BRL 43694 | **250** |
| 5-HT₄ | h | [³H]-GR 113808 | **> 1,000** |
| 5-HT₅ | h | [³H]-LSD | **100** |
| 5-HT₈ | h | [³H]-LSD | **> 1,000** |
| 5-HT₇ | h | [³H]-LSD | **3.2** |
| 5-HTᵣₑᵤₚₜₐₖₑ | h | [³H]-paroxetine | **> 1,000** |
| | | | |
| α₁-adrenergic | r | [³H]-prazosin | **> 1,000** |
| α_{1A}-adrenergic | r | [³H]-prazosin | **630** |
| α_{1B}-adrenergic | r | [³H]-prazosin | **> 1,000** |
| α₂-adrenergic | r | [³H]-RX 821002 | > **1,000** |
| β₁-adrenergic | h | [³H]-CGP 12177 | **50** |
| β₂-adrenergic | h | [³H]-CGP 12177 | **40** |
| β₃-adrenergic | h | [¹²⁵I]-iodocyanopindolol | **> 1,000** |
| NAᵣₑᵤₚₜₐₖₑ | h | [³H]-nisoxetin | **> 1,000** |
| | | | |
| Dopamine-**D₁** | h | [³H]-SCH 23390 | **> 1,000** |
| Dopamine-**D₂** | h | [³H]-spiperone | **> 1,000** |
| Dopamine-**D₃** | h | [³H]-spiperone | **> 1,000** |
| Dopamine-**D₄** | h | [³H]-spiperone | **> 1,000** |
| Dopamine-**D₅** | h | [³H]-SCH 23390 | **> 1,000** |
| Dopamineᵣₑᵤₚₜₐₖₑ | h | [³H]-GBR 12935 | **> 1,000** |
| | | | |
| Muscarine-**M₁** | h | [³H]-pirenzepine | **> 1,000** |
| Muscarine-**M₂** | h | [³H]-AFDX-384 | **> 1,000** |
| Muscarine-**M₃** | h | [³H]-4-DAMP | **> 1,000** |
| Muscarine-**M₄** | h | [³H]-4-DAMP | **> 1,000** |
| Muscanne-**M₅** | h | [³H]-4-DAMP | **> 1,000** |
| | | | |
| Histamine-**H₁** | h | [³H]-pyrilamine | **> 1,000** |
| Histamine-**H₂** | h | [¹I]-APT | **> 1,000** |
| Histamine-**H₃** | r | [³H]-α-methythistamine | **> 10,000** |
| | | | |
| tryptamine | r | [³H]-tryptamine | **> 10,000** |
| melatonin | c | [¹²⁵I]-2-iodomelatonin | **> 10,000** |
| nicotine | r | [³H]-cytisine | **> 10,000** |
| | | | |
| µ-opiate | r | [³H]-DAMGO | **> 1,000** |
| κ-opiate | r | [³H]-U 69593 | **> 1,000** |
| δ-opiate | r | [³H]-DPDPE | **> 1,000** |
| nociceptin (ORL₁) | h | [³H]-nociceptine | **> 1,000** |
| sigma | r | [³H]-DTG | **> 1,000** |
| sigma-SG₁ | g | [³H]-pentazocone | **> 1,000** |
| sigma-SG₂ | r | [³H]-DTG | **> 1,000** |
| | | | |
| cannabinoid-CB₁ | h | [³H]-WIN 55,212-2 | **> 10,000** |
| | | | |
| Ca⁺⁺-channel | p | [³H]-fluspirilene | **> 10,000** |
| Ca⁺⁺-channel | r | [³H]-nitrendipine | **> 10,000** |
| Ca⁺⁺-channel | r | [³H]-diltiazem | **> 10,000** |
| Ca⁺⁺-channel | r | [¹²⁵I]-Ω-conotoxin | **> 1,000** |
| Ca⁺⁺-channel | r | [³H]-D-888 | **130** |
| Ca⁺⁺-channel | r | [³H]-devapamil | **> 10,000** |
| Na⁺-channel | r | [³H]-bathrachotoxinin | **> 10,000** |
| K⁺-channel | r | [¹²⁵I]-α-dendrotoxin | **> 1,000** |
| K⁺-channel | r | [¹²⁵I]-apamin | **> 1,000** |
| | | | |
| Adenosine-**A₁** | h | [³H]-DPCPX | **> 1,000** |
| Adenosine-**A_{2A}** | h | [³H]-CGS 21680 | **> 1,000** |
| Adenosine-**A₃** | h | [³H]-AB-MECA | **> 1,000** |
| Purine-**P2X** | r | [³H]-ab-MeATP | **> 1,000** |
| GABA_{A} | r | [³H]-muscimol | **> 10,000** |
| GABA_{B} | r | [³H]-PK 11195 | **> 1,000** |
| Glycine | r | [³H]-strychnine | **> 10,000** |
| Glycine _{strychn.ins}. | r | [³H]-MDL105519 | **> 10,000** |
| NMDA | r | [³H]-CGS 19755 | **> 10,000** |
| | | | |
| angiotensin-**AT1** | h | [¹²⁵I]-angiotensin II | **> 1,000** |
| angiotensin-**AT2** | h | [¹²⁵I]-CPG 42112A | **> 1,000** |
| benzodiazepine | r | [³H]-diazepam | **> 10,000** |
| bombesin | r | [¹²⁵I]-bombesin | **> 1,000** |
| bradykinin | h | [³H]-bradykinin | **> 1,000** |
| CC**K_{A}** | h | [³H]-devazepide | **> 1,000** |
| CC**K_{B}** | h | [³H]-CCKB | **> 1,000** |
| CC**R1** | h | [¹²⁵I]-MIP-1a | **> 1,000** |
| CG**RP** | h | [¹²⁵I]-CGRPa | **> 1,000** |
| CR**F** | h | [¹²⁵I]-oCRF | **> 10,000** |
| Endothelin-**ET_{A}** | h | [¹²⁵I]-endothelin-**1** | **> 1,000** |
| Endothelin-**ET_{B}** | h | [¹²⁵I]-endothelin-**1** | **> 1,000** |
| Galanin-**GAL₁** | h | [¹²⁵I]-galanin | **> 1,000** |
| Galanin-**GAL₂** | h | [¹²⁵I]-galanin | **> 1,000** |
| Interleukine-**6** | h | [¹²⁵I]-interleukine-6 | **> 10,000** |
| Interleukine-**8** | h | [¹²⁵I]-interleukine-8 | **> 1,000** |
| LTB₄ | g | [³H]-LTB₄ | **> 10,000** |
| LTD₄ | g | [³H]-LTD₄ | **> 10,000** |
| melanocortin | h | [¹²⁵I]-NDP-a-MSH | **> 1,000** |
| Neurokinin-**NK₁** | h | [³H]-substance P | **> 1,000** |
| Neurokinin-**NK₂** | h | [¹²⁵I]-neurokinin_{A} | **> 1,000** |
| Neurokinin-**NK₃** | h | [³H]-SR 142801 | **> 1,000** |
| Neuropeptide **Y₁** | h | [¹²⁵I]-PYY | **> 1,000** |
| Neuropeptide **Y₂** | h | [¹²⁵I]-PYY | **> 1,000** |
| Neurotensin-**NT₁** | h | [¹²⁵I]-neurotensin | **> 1,000** |
| PACAP | r | [¹²⁵I]-PACAP 1-27 | **> 1,000** |
| Prostaglandin-**I₂** | h | [³H]-iloprost | **> 1,000** |
| Prostaglandin | h | [³H]-SQ 29548 | **> 1,000** |
| somatostatin | m | [¹²⁵I]-somatostatin | **> 1,000** |
| TRH | r | [³H]-TRH | **> 10,000** |
| Tumor necrosis f. | r | [¹²⁵I]-TNFα | **> 1,000** |
| Vasopressine-**V_{1A}** | h | [³H]-vasopressine | **> 1,000** |
| VIP₁ | h | [¹²⁵I]-VIP | **> 1,000** |

| | | | |
|---|---|---|---|
| ***S¹: b** = bovine, **c** = chicken, **g**= guines pig, **h** = human, **m** = mouse, **p** = pig: **r** = **rat*** | | | |

## Claims

1. Use of 3-amino-8-(1-piperazinyl)-2H-1-benzopyran-2-one, having formula (2): 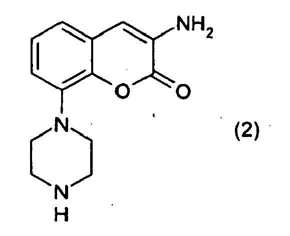 and all stereoisomers and pharmacologically acceptable salts thereof, for the preparation of a pharmaceutical composition for the treatment, amelioration or prevention of Parkinson's disease, Huntington's Chorea, progressive supranuclear palsy, Wilson's disease, Tourette's syndrome, symptomatic and non-symptomatic epilepsy, seizures, including refractory seizures and post-stroke seizures and other electroconvulsive disorders, various chronic tremors, including essential tremor, tics and dystonias.

2. Use as claimed in claim 1, wherein the pharmacologically acceptable salt is the mono hydrochloric acid mono hydrate.

3. Use as claimed in claim 1 or claim 2, wherein said symptomatic and non-symptomatic epilepsy are benign occipital epilepsy, benign rolandic epilepsy, frontal lobe epilepsy, occipital lobe epilepsy, mesial temporal lobe epilepsy and parietal lobe epilepsy; generalized idiopathic epilepsies including benign myoclonic epilepsy in infants, juvenile myoclonic epilepsy, childhood absence epilepsy, juvenile absence epilepsy and epilepsy with generalized tonic clonic seizures in childhood; generalized symptomatic epilepsies including infantile spasm (West syndrome), Lennox-Gastaut syndrome and progressive myoclonus epilepsies, and unclassified epilepsies including refractory epilepsy, post-stroke epilepsy, febrile fits, epilepsy with continuous spike and waves in slow wave sleep, Landau Kleffner syndrome, Rasmussen's syndrome and epilepsy and inborn errors of metabolism.

4. The mono hydrochloric acid mono hydrate of 3-amino-8-(1-piperazinyl)-2H-1-benzopyran-2-one for use in the treatment, amelioration or prevention of Parkinson's disease, Huntington's Chorea, progressive supranuclear palsy, Wilson's disease, Tourette's syndrome, symptomatic and non-symptomatic epilepsy, seizures, including refractory seizures and post-stroke seizures and other electroconvulsive disorders, various chronic tremors, including essential tremor, tics and dystonias.

5. The mono hydrochloric acid mono hydrate of 3-amino-8-(1-piperazinyl)-2H-1-benzopyran-2-one for use in the treatment, amelioration or prevention of benign occipital epilepsy, benign rolandic epilepsy, frontal lobe epilepsy, occipital lobe epilepsy, mesial temporal lobe epilepsy and parietal lobe epilepsy; generalized idiopathic epilepsies including benign myoclonic epilepsy in infants, juvenile myoclonic epilepsy, childhood absence epilepsy, juvenile absence epilepsy and epilepsy with generalized tonic clonic seizures in childhood; generalized symptomatic epilepsies including infantile spasm (West syndrome), Lennox-Gastaut syndrome and progressive myoclonus epilepsies, and unclassified epilepsies including refractory epilepsy, post-stroke epilepsy, febrile fits, epilepsy with continuous spike and waves in slow wave sleep, Landau Kleffner syndrome, Rasmussen's syndrome and epilepsy and inborn errors of metabolism.

## Patentansprüche

1. Verwendung von 3-Amino-8-(1-piperazinyl)-2H-1-benzopyran-2-on der Formel (2): 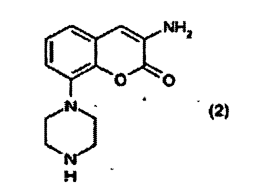 und aller Stereoisomere und pharmakologisch akzeptablen Salze davon, zur Herstellung einer pharmazeutischen Zusammensetzung zur Behandlung, Linderung oder Prävention von Parkinson'schen Krankheit, Huntington-Chorea, progressiver supranuklearer Blickparase, Morbus Wilson, Tourette-Syndrom, symptomatischer und nicht symptomatischer Epilepsie, Anfällen, einschließlich refraktärer Anfälle und Anfälle nach Schlaganfällen und anderer Elektrokrampfstörungen, verschiedenen chronischen Tremoren, einschließlich essentieller Tremor, Ticks und Dystonien.

2. Verwendung nach Anspruch 1, wobei das pharmakologisch akzeptable Salz Mono-HCl-Monohydrat ist.

3. Verwendung nach Anspruch 1 oder 2, wobei die symptomatische oder nicht symptomatische Epilepsie ist: gutartige okzipitale Epilepsie, gutartige Rolando-Epilepsie, Stirnlappen-Epilepsie, Okzipitallappen-Epilepsie, mesiale Temporallappen-Epilepsie und Parietallappen-Epilepsie; generalisierte idiopathische Epilepsien, einschließlich gutartiger myoklonischer Epilepsie bei Kleinkindern, myoklonischer Epilepsie bei Jugendlichen, Pyknolepsie bei Kindern, Pyknolepsie bei Jugendlichen und Epilepsie mit generalisierten tonisch-klonischen Anfällen im Kindesalter; generalisierte symptomatische Epilepsien, einschließlich Spasmus bei Kleinkindern (West-Syndrom), Lennox-Gastaut-Syndrom und progressiver myoklonischer Epilepsien; und unklassifizierte Epilepsien, einschließlich refraktärer Epilepsie, Epilepsie nach Schlaganfällen, Fieberanfälle, Epilepsie mit kontinuierlichen Spitzen und Wellen im "Slow-Wave"-Schlaf, Landau-Kleffner-Syndrom, Rasmussen-Syndrom und Epilepsie und angeborene Stoffwechselstörungen.

4. Mono-HCl-Monohydrat von 3-Amino-8-(1-piperazinyl)-2H-1-benzopyran-2-on zur Verwendung bei der Behandlung, Linderung oder Prävention von Parkinson'schen Krankheit, Huntington-Chorea, progressiver supranuklearer Blickparase, Morbus Wilson, Tourette-Syndrom, symptomatischer und nicht symptomatischer Epilepsie, Anfällen, einschließlich refraktärer Anfälle und Anfälle nach Schlaganfällen und anderer Elektrokrampfstörungen, verschiedenen chronischen Tremoren, einschließlich essentieller Tremor, Ticks und Dystonien.

5. Mono-HCl-Monohydrat von 3-Amino-8-(1-piperazinyl)-2H-1-benzopyran-2-on zur Verwendung bei der Behandlung, Linderung oder Prävention von gutartiger okzipitaler Epilepsie, gutartiger Rolando-Epilepsie, Stirnlappen-Epilepsie, Okzipitallappen-Epilepsie, mesialer Temporallappen-Epilepsie und Parietallappen-Epilepsie; generalisierten idiopathischen Epilepsien, einschließlich gutartiger myoklonischer Epilepsie bei Kleinkindern, myoklonischer Epilepsie bei Jugendlichen, Pyknolepsie bei Kindern, Pyknolepsie bei Jugendlichen und Epilepsie mit generalisierten tonisch-klonischen Anfällen im Kindesalter; generalisierten symptomatischen Epilepsien, einschließlich Spasmus bei Kleinkindern (West-Syndrom), Lennox-Gastaut-Syndrom und progressiver myoklonischer Epilepsien; und unklassifizierten Epilepsien, einschließlich refraktärer Epilepsie, Epilepsie nach Schlaganfällen, Fieberanfällen, Epilepsie mit kontinuierlichen Spitzen und Wellen im "Slow-Wave"-Schlaf, Landau-Kleffner-Syndrom, Rasmussen-Syndrom und Epilepsie und angeborener Stoffwechselstörungen.

## Revendications

1. Utilisation de la 3-amino-8-(1-pipérazinyl)-2H-1-benzopyran-2-one, ayant la formule (2) : 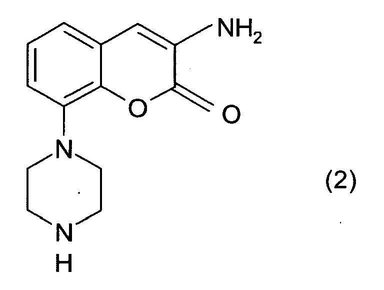 et de tous ses stéréoisomères et sels pharmacologiquement acceptables pour la préparation d'une composition pharmaceutique pour le traitement, l'amélioration ou la prévention de la maladie de Parkinson, de la chorée de Huntington, de la paralysie supranucléaire progressive, de la maladie de Wilson, du syndrome de Tourette, de l'épilepsie symptomatique et non symptomatique, des crises, incluant les crises réfractaires et les crises post-attaque et d'autres troubles électroconvulsifs, de différents tremblements chroniques, incluant le tremblement essentiel, des tics et des dystonies.

2. Utilisation selon la revendication 1 où le sel pharmacologiquement acceptable est le monochlorhydrate monohydraté.

3. Utilisation selon la revendication 1 ou 2 où lesdites épilepsies symptomatiques et non symptomatiques sont l'épilepsie occipitale bénigne, l'épilepsie rolandique bénigne, l'épilepsie du lobe frontal, l'épilepsie du lobe occipital, l'épilepsie du lobe temporal mésial et l'épilepsie du lobe pariétal ; les épilepsies idiopathiques généralisées incluant l'épilepsie myoclonique bénigne chez les jeunes enfants, l'épilepsie myoclonique juvénile, l'épilepsie d'absence chez l'enfant, l'épilepsie d'absence juvénile et l'épilepsie avec crises cloniques toniques généralisées chez l'enfant ; les épilepsies symptomatiques généralisées incluant le spasme infantile (syndrome de West), le syndrome de Lennox-Gastaut et les épilepsies myocloniques progressives, et les épilepsies non classées incluant l'épilepsie réfractaire, l'épilepsie post-attaque, les accès fébriles, l'épilepsie avec des pointes et ondes continues dans le sommeil à ondes lentes, le syndrome de Landau Kleffner, le syndrome de Rasmussen et l'épilepsie et les erreurs innées du métabolisme.

4. Monochlorhydrate monohydraté de la 3-amino-8-(1-pipérazinyl)-2H-1-benzopyran-2-one destiné à être utilisé dans le traitement, l'amélioration ou la prévention de la maladie de Parkinson, de la chorée de Huntington, de la paralysie supranucléaire progressive, de la maladie de Wilson, du syndrome de Tourette, de l'épilepsie symptomatique et non symptomatique, des crises, incluant les crises réfractaires et les crises post-attaque et d'autres troubles électroconvulsifs, de différents tremblements chroniques, incluant le tremblement essentiel, des tics et des dystonies.

5. Monochlorhydrate monohydraté de la 3-amino-8-(1-pipérazinyl)-2H-1-benzopyran-2-one destiné à être utilisé dans le traitement, l'amélioration ou la prévention de l'épilepsie occipitale bénigne, l'épilepsie rolandique bénigne, l'épilepsie du lobe frontal, l'épilepsie du lobe occipital, l'épilepsie du lobe temporal mésial et l'épilepsie du lobe pariétal ; des épilepsies idiopathiques généralisées incluant l'épilepsie myoclonique bénigne chez les jeunes enfants, l'épilepsie myoclonique juvénile, l'épilepsie d'absence chez l'enfant, l'épilepsie d'absence juvénile et l'épilepsie avec crises cloniques toniques généralisées chez l'enfant ; des épilepsies symptomatiques généralisées incluant le spasme infantile (syndrome de West), le syndrome de Lennox-Gastaut et les épilepsies myocloniques progressives, et des épilepsies non classées incluant l'épilepsie réfractaire, l'épilepsie post-attaque, les accès fébriles, l'épilepsie avec des pointes et ondes continues dans le sommeil à ondes lentes, le syndrome de Landau Kleffner, le syndrome de Rasmussen et l'épilepsie et les erreurs innées du métabolisme.
